# EUROPEAN PATENT SPECIFICATION

(11) **EP 4 355 470 B1**
(45) Date of publication and mention of the grant of the patent: **16.10.2024**
(21) Application number: 23768605.0
(22) Date of filing: 17.07.2023
(51) Int. Cl.: B01F 35/513, B01F 27/054, B01F 27/90, B01F 27/91, B33Y 80/00

(54) **IMPELLER SYSTEM FOR USE WITH A BIOREACTOR**
LAUFRADSYSTEM ZUR VERWENDUNG MIT EINEM BIOREAKTOR
SYSTÈME DE TURBINE DESTINÉ À ÊTRE UTILISÉ AVEC UN BIORÉACTEUR

(30) Priority: 14.07.2022 US 202263368377 P; 24.03.2023 NL 2034416
(43) Date of publication of application: 24.04.2024
(73) Proprietor: APPLIKON BIOTECHNOLOGY B.V., 2629 JG Delft (NL)
(72) Inventor: KERKLAAN, Jeroen Johannes Theodorus, 2629 JG DELFT (NL); WALVOORT, Wilbert Timotheüs, 2629 JG DELFT (NL)
(74) Representative: Algemeen Octrooi- en Merkenbureau B.V.
(86) International application number: PCT/IB2023/000432
(87) International publication number: WO 2024/013569

(56) References cited:
- DE-U1- 20 000 468
- FR-A- 366 804
- US-A1- 2010 260 010

## Description

### FIELD

The present disclosure relates to an impeller system for use with a bioreactor, a method of using such an impeller system, as well as a method of manufacturing such an impeller system.

### BACKGROUND

WO 2013/151733 A1 discloses a fluid mixing system including a container, such as a flexible bag, bounding a compartment. A flexible drive line is disposed within the compartment, the drive line having a first end rotatably connected to a first end of the container and an opposing second end rotatably connected to a second end of the container. At least one mixing element, such as an impeller, is coupled with the flexible drive line. Rotation of the drive line facilitates rotation of the impeller within the container. The impeller may comprise pivotable or foldable blades. By folding the blades (whereas the blade bends about location between the axis and the blade tip), the container can be more fully collapsed around the impeller while minimizing risk of damage to the container and to the blades, for instance during transportation. Each of the blades may catch the fluid and automatically move from a collapsed (folded) position to an expanded position (unfolded) for mixing the fluid.

However, a problem with the fluid mixing system of WO 2013/151733 A1 is that in the collapsed state the foldable blades still occupy quite a lot of space around the drive line.

Furthermore, relatively complex mechanical systems are required to unfold the foldable blades to the expanded position in order to start agitation.

An object of the present disclosure is thus to provide an impeller system for use with a bioreactor, wherein in the collapsed state the blades are collapsed towards each other to occupy less space around the drive line.

A further object of the disclosure is to provide an impeller system for use with a bioreactor, wherein relatively less complex mechanical systems are required to transition the collapsed blades to an un-collapsed position in order to start agitation.

### SUMMARY

According to embodiments of the present disclosure, an impeller system for use with a bioreactor is provided, comprising:
- a drive shaft configured for being rotated in a rotational direction around a longitudinal axis of the drive shaft by a drive motor of the bioreactor;
- at least two impeller blades connected to the drive shaft, configured for being rotated along with the drive shaft when the drive shaft is rotated, wherein the at least two impeller blades are configured for transitioning from
   a collapsed state, wherein the at least two impeller blades are not positioned axisymmetrically around the drive shaft, such as being aligned along the longitudinal axis, to
   an un-collapsed state, for performing agitation, wherein the at least two impeller blades are positioned axisymmetrically around the drive shaft,
   wherein at least one of the at least two impeller blades transitions from the collapsed state to the un-collapsed state by rotating along a circumference of the drive shaft due to resistance from a liquid in the bioreactor when agitation is performed.

Due to the at least two impeller blades, such as three, four, five, six, or more blades, preferably all blades, not being positioned axisymmetrically around the drive shaft, such as being aligned along the longitudinal axis, in the collapsed state, much less space is occupied around the drive shaft in the collapsed state, allowing for easier transportation and a greatly reduced size of the impeller system when e.g. a bioreactor bag or a similar flexible container for bioreaction is used, i.e. when the flexible container for bioreaction is collapsed around the impeller system.

In the context of the present patent application, "aligned" means "substantially radially aligned", i.e. the angle formed between similar radially extending features of a first item (such as a blade) and a second item (such as an adjacently radially disposed blade) is less than 60 degrees from each other, more preferably less than 45 degrees from each other, and even more preferably less than 30 degrees from each other, and even more preferably less than 20, degrees from each other, and even more preferably less than 15 degrees from each other about a common axis of rotation of the drive shaft, such as the longitudinal axis of the drive shaft, especially the portion of the drive shaft in which the blades are connected or otherwise engage the drive shaft.

Furthermore, the at least one of the at least two impeller blades transitions along the circumference of the drive shaft (e.g., along a circumferential arc) from the collapsed state to the un-collapsed state by rotating along a circumference of the drive shaft due to resistance from a liquid in the bioreactor when agitation is performed. Therein, at least one of the two (or more) impeller blades "automatically" and gradually transitions to its own individual/unique position along the circumference of the drive shaft in the final axisymmetric configuration of the impeller blades in the un-collapsed state, basically in a very natural way: the hydrodynamic forces exerted by the liquid on the at least one of the at least two impeller blades pushes the at least one of the impeller blades into its unique place in the final axisymmetric configuration of the impeller blades in the un-collapsed state, for instance by causing/allowing the at least one of the at least two impeller blades to slide (e.g., rotationally slide) along the circumference (or a circumferential arc) of the drive shaft. Thus, the use of complex mechanical systems to unfold the impeller blades can be avoided.

A further advantage of the above impeller system is that in the collapsed state, the at least two impeller blades are less likely to damage the flexible container (e.g., pliable bag) for bioreaction when the flexible container for bioreaction is collapsed around the impeller system, because the at least two impeller blades are aligned along the longitudinal axis, thereby distributing any pressure exerted on the inside of the (respective side of the) flexible container for bioreaction over multiple impeller blades, thus preventing puncturing or tearing of the flexible container for bioreaction.

Further general state of the art which is not considered to be of particular relevance to the present disclosure is disclosed in FR 366 804 A, US 2010/260010 A1 and DE 200 00 468 U1: FR 366 804 A discloses a mixing device as per the preamble of claim 1 for mixing beer, wine or other liquids in a barrel. US 2010/260010 A1 discloses a container with a flexible container wall, in particular a disposable bioreactor, with a container interior in which a mixer is arranged at one end of a mixer shaft which is passed through the container wall and is drivable from the outside, wherein the mixer shaft and/or mixer is/are designed such that it/they are foldable and such that it/they can be stabilized from the outside. DE 200 00 468 U1 discloses a stirring device for homogenizing manure, with a drive shaft and agitator blades, which are mounted on the drive shaft so that they can be folded about pivot axes, the agitator blades being aligned parallel to the drive shaft when at a standstill and a position at an angle to the drive shaft when the drive shaft is rotating.

An embodiment relates to an aforementioned impeller system, wherein the at least two impeller blades are each independently connected to the drive shaft. Thus, the at least one individual impeller blade can move independently from the other impeller blades to the respective impeller blade's final position in the un-collapsed state, increasing reliability of the impeller system. Furthermore, the foregoing allows "tuning" of the impeller system by e.g. removing or adding impeller blades, which also facilitates manufacturing.

An embodiment of the present disclosure relates to an aforementioned impeller system, wherein at least one of the at least two impeller blades is independently attached to the drive shaft with a ring, configured for rotation around the drive shaft, wherein the ring is configured for rotating from a first orientation on the drive shaft in the collapsed state to a second, individual orientation on the drive shaft in the un-collapsed state, such that the at least two impeller blades are positioned axisymmetrically around the drive shaft. The use of such rotatable rings allows for a natural movement of the at least one of the at least two impeller blades to its final position in the un-collapsed state. Removal or addition of impeller blades is also further simplified.

An embodiment relates to an aforementioned impeller system, wherein one of the ring or a local circumference of the drive shaft at the axial location of the ring is provided with an engagement portion and the other of the ring or the local circumference is provided with an engagement member, wherein the engagement portion and the engagement member are configured to engage each other when the ring has reached the second orientation, thereby preventing rotation of the ring past the second orientation.

An embodiment relates to an aforementioned impeller system, wherein, in the collapsed state, the at least two impeller blades are adjacent to each other along the longitudinal axis. Thus, by spacing the at least two impeller blades closely together, such as by stacking them onto each other along the longitudinal axis, chances of local pressure points occurring further decrease, thus further preventing puncturing or tearing of the flexible container for bioreaction.

An embodiment relates to an aforementioned impeller system, wherein, in the collapsed state, the at least two impeller blades are adjacent to each other along the longitudinal axis in such a way, that contours of the at least two impeller blades are aligned or otherwise can be brought into approximation of each other, when viewed along the longitudinal axis. Thus, a compact "package" of impeller blades is achieved, wherein the at least two impellers are prevented from protruding with respect to each other in a direction perpendicular to the longitudinal axis due to the contours of the impeller blades being aligned in the longitudinal direction, thus further preventing local puncturing of the flexible container for bioreaction.

An embodiment relates to an aforementioned impeller system, wherein radially outer edges of the two or more impeller blades are rounded in a main plane of the impeller blade, allowing the bioreactor be smoothly collapsed over the impeller blades, for instance when transporting or storing the flexible container for bioreaction and the impeller system, further decreasing the risk of damage occurring to the flexible container for bioreaction.

An embodiment relates to an aforementioned impeller system, wherein the rounded, radially outer edges of the two or more impeller blades have a constant radius of curvature, to further facilitate smooth arrangement of the flexible container for bioreaction over the radially outer edges of the impeller blades in the collapsed state.

An embodiment relates to an aforementioned impeller system, wherein radially outer edges of the two or more impeller blades are rounded in a plane transversal to the main plane of the impeller blade and the radially outer edges. Thus, the impeller blades are less "sharp" to further prevent puncturing or cutting of the flexible container for bioreaction in the collapsed state.

An embodiment relates to an aforementioned impeller system, wherein the at least two impeller blades in the collapsed state are aligned along the longitudinal axis, providing for a compact impeller blade package.

An embodiment relates to an aforementioned impeller system, wherein the at least two impeller blades in the collapsed state establish a rotational angle with respect to each other about the longitudinal axis that is less than 45 degrees, more preferably less than 30 degrees, and even more preferably less than 15 degrees, most preferably around 0 degrees, such that the impeller blades are close together in the rotational direction in the collapsed state.

An embodiment relates to an aforementioned impeller system, wherein the at least two impeller blades are each independently connected to the drive shaft, allowing for optimal design and operational flexibility.

Another aspect of the disclosure concerns a flexible container for bioreaction, comprising an aforementioned impeller system, wherein the impeller system is arranged inside the flexible container for bioreaction.

An embodiment relates to an aforementioned flexible container for bioreaction, wherein the at least two impeller blades are in the collapsed state.

An embodiment relates to an aforementioned flexible container for bioreaction, wherein the inside of the flexible container for bioreaction is sterile to a sterility assurance level of at least 10-3 SAL.

An embodiment relates to an aforementioned flexible container for bioreaction, further comprising a sterility barrier encapsulating the flexible container, wherein the sterility barrier is optionally configured as a bag, pouch, or a tub with a sealed lid.

Another aspect of the disclosure concerns a bioreactor, comprising a drive motor and an aforementioned impeller system or an aforementioned flexible container for bioreaction, wherein the drive shaft is connected to the drive motor.

Another aspect of the disclosure concerns a method of using an aforementioned impeller system, comprising the steps of:
- connecting the drive shaft to the drive motor of the bioreactor; and
- rotating the drive shaft around the longitudinal axis of the drive shaft by the drive motor of the bioreactor, wherein the at least one of the at least two impeller blades transitions from the collapsed state to the un-collapsed state by rotating along a circumference of the drive shaft due to resistance from the liquid in the bioreactor, for performing agitation of the liquid.

Another aspect of the disclosure concerns a method of manufacturing an aforementioned impeller system, comprising the step of:
- manufacturing a drive shaft configured for being rotated in a rotational direction around a longitudinal axis of the drive shaft by a drive motor of the bioreactor;
- manufacturing at least two impeller blades for connection to the drive shaft, and for being rotated along with the drive shaft, wherein at least one of the at least two impeller blades is configured for transitioning from
   a collapsed state, wherein the at least two impeller blades are not positioned axisymmetrically around the drive shaft, to
   an un-collapsed state, for performing agitation, wherein the at least two impeller blades are positioned axisymmetrically around the drive shaft,
   wherein the at least one of the at least two impeller blades transitions from the collapsed state to the un-collapsed state by rotating along a circumference of the drive shaft due to resistance from a liquid in the bioreactor when agitation is performed; and
- connecting the at least two impeller blades to the drive shaft.

An embodiment relates to an aforementioned method of manufacturing, wherein manufacturing the at least two impeller blades comprises 3D-printing at least one, such as two, of the at least two impeller blades. 3D-printing is any of various processes in which material is joined or solidified to create a three-dimensional object, with material being added together (such as liquid molecules or powder grains being fused together). 3D-printing is often used in both rapid prototyping and additive manufacturing (AM). Objects can be of almost any shape or geometry and typically are produced using digital model data from a 3D model or another electronic data source such as an Additive Manufacturing File (AMF) file (usually in sequential layers). There are many different technologies, like stereo-lithography (SLA) or fused deposit modeling (FDM). Thus, unlike material removed from a stock in the conventional machining process, 3D printing or AM builds a three-dimensional object from computer-aided design (CAD) model or AMF file, usually by successively adding material layer by layer.

An embodiment relates to an aforementioned method of manufacturing, wherein in the collapsed state the at least two impeller blades are aligned along the longitudinal axis.

### BRIEF DESCRIPTION OF THE DRAWINGS

The embodiments of the disclosure will be explained in more detail below, with reference to illustrative embodiments shown in the drawings. Therein:
Figure 1 shows an example embodiment of a bioreactor, comprising a drive motor and a flexible container for bioreaction with an impeller system according to the disclosure, wherein the drive shaft is connected to the drive motor;
Figure 2 shows an example embodiment of an impeller system according to the disclosure, such as the impeller system of Figure 1, with the impeller blades in the collapsed state;
Figure 3 shows an example embodiment of an impeller system according to the disclosure, such as the impeller system of Figure 1 or 2, with the impeller blades in the un-collapsed state;
Figure 4 shows an exploded view of an example embodiment of an impeller system according to the disclosure, such as the impeller system of Figure 1, 2 or 3, with the impeller blades in the un-collapsed state; and
Figures 5 - 7 show example embodiments of an impeller system according to the disclosure, comprising a connection mechanism for connecting a lower drive shaft portion to an upper drive shaft portion.

### DETAILED DESCRIPTION

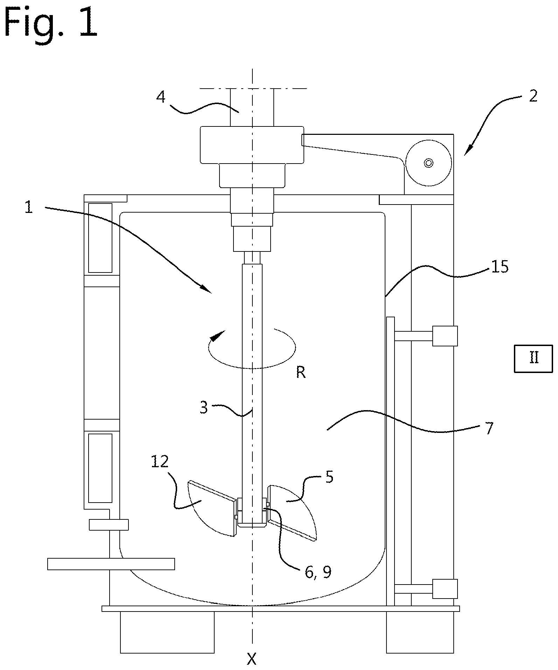

Figure 1 shows an example embodiment of a bioreactor 2, comprising a drive motor 4 and an impeller system 1 according to an example embodiment of the disclosure, wherein the drive shaft 3 is connected to the drive motor 4. The bioreactor 2 may be a single-use or multi-use bioreactor 2. The bioreactor 2 may be configured for an operational/work volume of 1 - 10.000 liters, preferably 10 - 5.000 liters, more preferably 50 - 3.000 liters, such as 40 - 60 liters. A bioreactor 2 generally relates to a manufactured or engineered device or system that supports a biologically active environment. The bioreactor 2 may be cylindrical and may be made of glass and/or stainless steel. The bioreactor 2 may also relate to a device or system designed to grow cells or tissues in the context of cell culture.

The impeller system 1 is arranged inside a flexible container for bioreaction 15. Outer surfaces of the flexible container for bioreaction 15, such as a bioreactor bag 15, are positioned against inner surfaces (i.e., inner sidewalls) of the bioreactor 2 to provide a proper fit, preferably without folds and the like. The flexible container for bioreaction 15 may be configured for single use. Such a single-use flexible container 15 has several advantages, in particular reducing assembly/disassembly, cleaning, sterilization and calibration demands. The impeller system 1 comprises the drive shaft 3, which is configured for being rotated in a rotational direction R around a longitudinal axis X of the drive shaft 3 by the drive motor 4 of the bioreactor 2. The drive shaft 3 may have a length of for instance 10 - 250 cm, such as 10 - 100 cm, for instance 10 - 50 cm, depending on the bioreactor 2 design. At least two impeller blades 5, such as two, three, four, fix, six or even more, are connected to the drive shaft 3, and are configured for being rotated along with the drive shaft 3 in the rotational direction R. The at least two impeller blades 5 are preferably arranged at a free end of the drive shaft 3, although other arrangements are also conceivable (e.g., such as being spaced from the free end of the drive shaft 3). The impeller blades 5 may have the form of a (flat) plate, although other shapes are also conceivable such as curved blades. The impeller blades 5 may also be arranged at an angle with respect to (a plane transversal to) the longitudinal axis X. The at least two impeller blades 5 are configured for transitioning from a collapsed state I, wherein the at least two impeller blades 5 are adjacent to each other or are otherwise capable brought into approximation of each other about the longitudinal axis X, to an un-collapsed state II, for performing agitation, wherein the at least two impeller blades are positioned rotationally away from each other axisymmetrically around the drive shaft 3. If two impeller blades 5 are used, in the un-collapsed state the blades would be radially spaced about the axis X from each other by about 180 degrees, wherein if three impeller blades 5 are used, in the un-collapsed state the blades would be radially spaced about the axis X by about 120 degrees. Figure 1 shows the impeller blades 5 in the un-collapsed state II. The at least one of the at least two impeller blades 5 transitions from the collapsed state I to the un-collapsed state II by rotating, i.e. moving, along a circumference 6 of the drive shaft 3 due to resistance from a liquid 7 in the bioreactor 2 when agitation is performed.

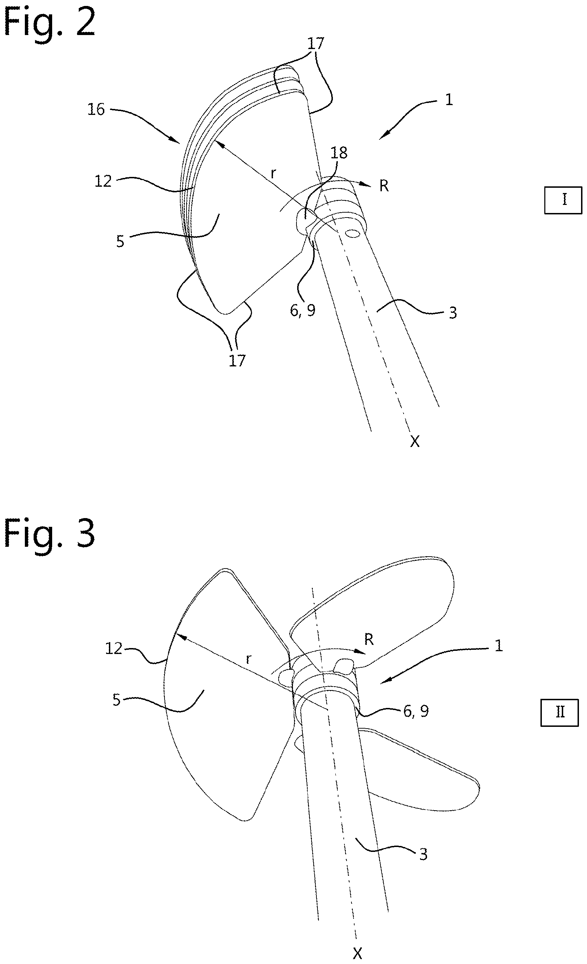

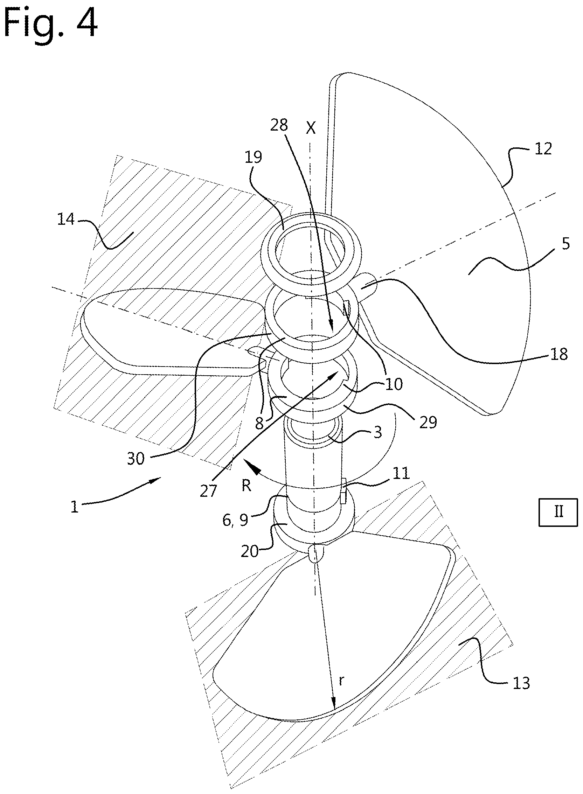

Figure 2 shows an example embodiment of an impeller system 1 according to the disclosure, such as the impeller system 1 of Figure 1, with the impeller blades 5 in the collapsed state I. The at least two impeller blades 5 are in the collapsed state I, e.g. for being stored or transported. The at least two impeller blades 5 may each be independently connected to the drive shaft 3, as will be more clearly explained with reference to Figure 4. In the collapsed state I, the at least two impeller blades 5 may be adjacent to each other 16 along the longitudinal axis X, forming a "package" of impeller blades 5, preferably in such a way, that contours 17 of the at least two impeller blades 5 are aligned or otherwise can be brought adjacent to each other in a contacting or non-contacting manner, when viewed along the longitudinal axis X. By example, radially adjacent blades can establish an angle to each other with respect to the longitudinal axis X that is less than 90 degrees, more preferably less than 60 degrees or 45 degrees, more preferably less than 40 degrees, preferably less than 30 degrees, preferably less than 15 degrees, preferably less than 10 degrees, preferably less than 5 degrees, and if geometrically feasible can establish an angle to each other at or about 0 degrees, all the foregoing subject to geometric constraints such as but not limited to their respective blade thickness, shape, means of connecting to the drive shaft, and/or longitudinal spacing along the drive shaft 3.

Figure 3 shows an example embodiment of an impeller system 1 according to the disclosure, such as the impeller system 1 of Figure 1 or 2, with the impeller blades 5 in the un-collapsed state I. The impeller blades 5 are now axisymmetrically arranged around the drive shaft 3, for performing agitation, and as shown using three blades, may establish an angle to each other with respect to the longitudinal axis X of about 120 degrees.

Figure 4 shows an exploded view of an example embodiment of an impeller system 1 according to the disclosure, such as the impeller system 1 of Figure 1, 2 or 3, with the impeller blades 5 in the un-collapsed state I. Radially outer edges 12 of the two or more impeller blades 5 are preferably rounded in a main plane 13 of the impeller blade 5. The rounded, radially outer edges 12 of the two or more impeller blades 5 preferably have a constant radius of curvature r. The radius of curvature r could be 2 - 10 cm, such as 2 - 5 cm. The at least one of the at least two rotatable impeller blades 5 may be independently attached to the drive shaft 3 with a rotatable ring 8, provided with an engagement portion 10, such as a lower rotatable ring 29, respectively, and an upper rotatable ring 30, as shown in Figure 4.

The rotatable rings 8 may be configured for rotation around the drive shaft 3, wherein the rotatable rings 8 are configured for rotating from a first orientation (i.e. a first angular position) on the drive shaft 3 in the collapsed state I to a second orientation (i.e. a second angular position) on the drive shaft 3 in the un-collapsed state II, such that the at least two rotatable impeller blades 5 are positioned axisymmetrically around the drive shaft 3, with each of the impeller blades 5 having a unique axisymmetric position. As shown in Figure 4, the lower rotatable ring 29 may be provided with a lower circumferential recess 27, whereas the upper rotatable ring 30 may be provided with an upper circumferential recess 28. The local circumference 9 of the drive shaft 3 is provided with an engagement member 11 in the form of a notch, protrusion or the like. The circumferential length of the lower circumferential recess 27 differs from the circumferential length of the upper circumferential recess 28.

Essentially, the rings 8 act with respect to the drive shaft 3 as a keyed slot mechanism. Keyed slots are typically designed with little to no "slop" to prevent rotation due to the similar dimension of the key width and the slot width. In the embodiment shown in Figure 4, however, relatively large keyway widths, i.e. the circumferential lengths of the lower and upper circumferential recesses 27, 28, are used to cause large degrees of "slop" to permit additional rotation, and the amounts of rotation permitted are different because the keyway equivalents of the lower and upper rings 29, 30, i.e. the lengths of the lower and upper circumferential recesses 27, 28, are different in size for each ring 8. The "key" equivalent of the embodiment shown in Figure 4 is essentially the engagement member 11.

Each ring 8, i.e. each of the lower ring 29 and the upper ring 30, has a "keyway" (i.e. the respective circumferential lengths of the lower and upper circumferential recesses 27, 28) that is drastically larger than the key (engagement member 11), such that when the second impeller blade 5 associated with the lower ring 29 - when counted upwards from the lower end of the impeller system 2 of Figure 4 - rotates about the drive shaft 3 along the circumferential recess 27 and at the engagement portion 10 makes contact at one side of the engagement portion 10 with the engagement member 11, it stops rotating, and when the third impeller blade 5 associated with the upper ring 30, rotates about the drive shaft 3 along the upper circumferential recess 28 and the engagement portion 10 of the upper ring 30 makes contact with the same key, i.e. engagement member 11, it also stops rotating.

The lower ring 29 has a keyway (circumferential recess 27 length) that is so large that it permits rotation to at or about 120 degrees, and the upper ring 30 has a keyway (circumferential recess 28 length) that is so large that it permits rotation to at or about 240 degrees, so that if three impeller blades 5 are used they are placed at or about 120 degrees out of phase from each other about the rotational axis X.

The ring 8, such as the two rings 8 as shown in Figure 4, may be kept in their longitudinal position by using a lower end ring 20 and an upper end ring 19. The rings 8 are then firmly "locked" (i.e. longitudinally) between the upper end ring 19 and the lower end ring 20. As can be seen from Figure 4, the lower end ring 20 may be provided with an impeller blade 5 that is fixedly attached to the drive shaft 3, e.g. being integrally formed therewith, i.e. unable to move with respect to the local circumference 9 of the drive shaft 3. The other impeller blades 5 as shown in Figure 4, in contrast, are configured to rotate with respect to the drive shaft 3 due to the resistance of the liquid (i.e. in a direction opposite to the rotational direction R, as the skilled person will understand).

The radially outer edges 12 of the two or more impeller blades 5 are preferably rounded in a plane 14 transversal to the main plane 13 of the impeller blade and the radially outer edges 12.

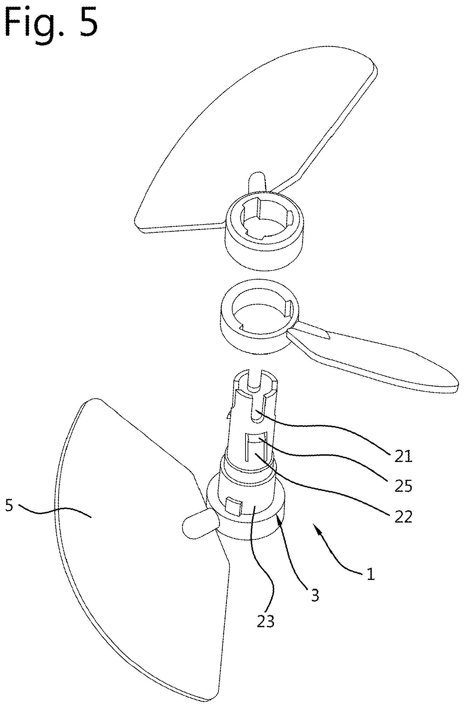

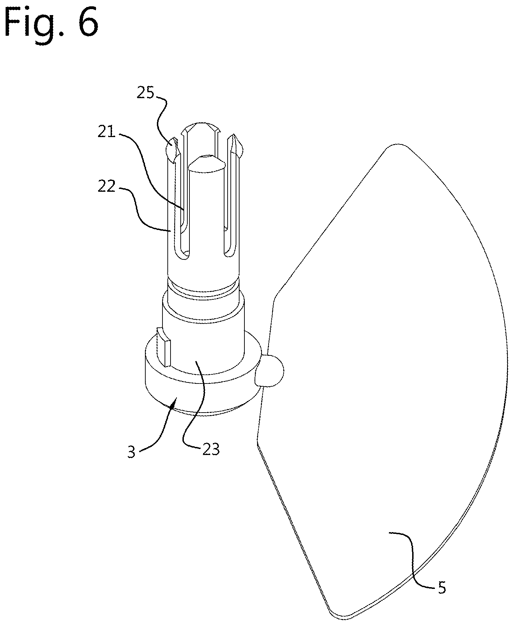

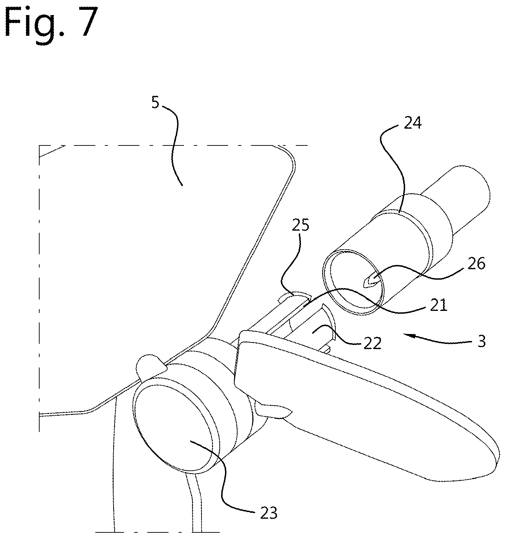

Figures 5 - 7 show example embodiments of an impeller system 1 according to the disclosure, comprising a connection mechanism for connecting a (during use) lower drive shaft portion 23 of the drive shaft 3 to an upper drive shaft portion 24 of the drive shaft 3 (as more clearly shown in Figure 7). The two or more impeller blades 5 are connected to the lower drive shaft portion 23. Figure 5 shows a first variant of the connection mechanism, wherein one or more longitudinal guiding grooves 21, such as one, two, three, four or more guiding grooves 21, are provided in the lower drive shaft portion 23, at an upper longitudinal end thereof. The guiding grooves 21 are configured for receiving one or more elongated guiding members 26, such as shown in Figure 7. Thus, the torque of a drive motor connected to the upper drive shaft portion 24 can be properly transmitted to the lower drive shaft portion 23. The first variant of the connection mechanism as shown in Figure 5 comprises relatively short guiding grooves 21 compared to the second variant of the connection mechanism shown in Figures 6 and 7, showing relatively longer guiding grooves 21.

The first variant, as shown in Figure 5, comprises one or more (radially) resilient locking members 22, arranged below the relatively short guiding grooves 21, for locking onto one or more corresponding protrusions (not shown) on the upper drive shaft portion 24. The upper drive shaft portion 24 may be hollow, such as shown in Figure 7, for receiving the lower drive shaft portion 23. The one or more protrusions may be arranged on an inside of a circumferential wall of such a hollow upper drive shaft portion 24. To facilitate locking "behind" such protrusions, one or more radially outwardly extending connection edges 25 may be provided on a longitudinally upper end of the resilient locking members 22.

The second variant, as shown in Figures 6 and 7, also comprises one or more (radially) resilient locking members 22 - although now arranged in between relatively longer guiding grooves 21, in the rotational/circumferential direction R, for locking onto one or more corresponding protrusions (not shown) on the upper drive shaft portion 24. The upper drive shaft portion 24 may be hollow, for receiving the lower drive shaft portion 23, as mentioned in the foregoing, such as shown in Figure 7. The resilient locking members 22 are basically alternating with the guiding grooves 21 in the rotational/circumferential direction R in the second variant. The one or more protrusions may again be arranged on an inside of a circumferential wall of such a hollow upper drive shaft portion 24. To facilitate locking behind the protrusions, one or more radially outwardly extending connection edges 25 may be provided on a longitudinally upper end of the resilient locking members 22. The skilled person will understand that features of the first and second variants can be combined or mixed, if desired.

As mentioned previously, another aspect of the disclosure relates to a method of using an aforementioned impeller system 1, comprising the steps of:
- connecting the drive shaft 3 to the drive motor 4 of the bioreactor 2; and
- rotating the drive shaft 3 around the longitudinal axis X of the drive shaft 3 by the drive motor 4 of the bioreactor 2, wherein the at least one of the at least two impeller blades 5 transitions from the collapsed state I to the un-collapsed state II by rotating along a circumference 6 of the drive shaft 3 due to resistance from the liquid 7 in the bioreactor, for performing agitation of the liquid 7.

Yet another aspect of the disclosure relates to a method of manufacturing an aforementioned impeller system 1, comprising the step of:
- manufacturing a drive shaft 3 configured for being rotated in a rotational direction R around a longitudinal axis X of the drive shaft 3 by a drive motor 4 of the bioreactor 2;
- manufacturing at least two impeller blades 5 for connection to the drive shaft 3, and for being rotated along with the drive shaft 3, wherein at least one of the at least two impeller blades 5 is configured for transitioning from
   a collapsed state I, wherein the at least two impeller blades 5 are not positioned axisymmetrically around the drive shaft 3, such as aligned along the longitudinal axis X of the drive shaft 3, to
   an un-collapsed state II, for performing agitation, wherein the at least two impeller blades 5 are positioned axisymmetrically around the drive shaft 3,
   wherein the at least one of the at least two impeller blades 5 transitions from the collapsed state I to the un-collapsed state II by rotating along a circumference 6 of the drive shaft 3 due to resistance from a liquid 7 in the bioreactor 2 when agitation is performed; and
- connecting the at least two impeller blades 5 to the drive shaft 3.

Manufacturing the at least two impeller blades 5 may comprise 3D-printing at least one of the at least two impeller blades 5.

It should be noted that in a preferred embodiment, at least one of the impeller blades 5, in particular a non-transitioning impeller blade 5 (i.e. an impeller blade 5 that does not rotate along a circumference of the drive shaft), may be rigidly connected or secured to the drive shaft 3, or optionally formed integrally with the drive shaft 3, during manufacture thereof, if desired.

### LIST OF REFERENCE NUMERALS

- 1.: Impeller system
- 2.: Bioreactor
- 3.: Drive shaft
- 4.: Drive motor
- 5.: Impeller blade
- 6.: Circumference of drive shaft
- 7.: Liquid
- 8.: Ring
- 9.: Local circumference
- 10.: Engagement portion
- 11.: Engagement member
- 12.: Radially outer edge
- 13.: Main plane of impeller blade
- 14.: Plane transversal to main plane and radially outer edges
- 15.: Bioreactor bag
- 16.: Stacked impeller blades
- 17.: Contour of impeller blade
- 18.: Pivot
- 19.: Upper end ring
- 20.: Lower end ring
- 21.: Guiding groove
- 22.: Resilient locking member
- 23.: Lower drive shaft portion
- 24.: Upper drive shaft portion
- 25.: Connection edge of resilient locking member
- 26.: Guiding member
- 27.: Circumferential recess of lower rotatable ring
- 28.: Circumferential recess of upper rotatable ring
- 29.: Lower rotatable ring
- 30.: Upper rotatable ring
- R: = Rotational direction
- X: = Longitudinal axis
- I: = Folded/collapsed state
- II: = Un-collapsed state
- r: = Radius of curvature

## Claims

1. Impeller system (1) for use with a bioreactor (2), comprising:
- a drive shaft (3) configured for being rotated in a rotational direction (R) around a longitudinal axis (X) of the drive shaft by a drive motor (4) of the bioreactor;
- at least two impeller blades (5) connected to the drive shaft, configured for being rotated along with the drive shaft when the drive shaft is rotated, wherein the at least two impeller blades are configured for transitioning from
a collapsed first state (I), wherein the at least two impeller blades are not positioned axisymmetrically around the drive shaft, to
an un-collapsed state (II), for performing agitation, wherein the at least two impeller blades are positioned axisymmetrically around the drive shaft,
**characterized in that** at least one of the at least two impeller blades transitions from the collapsed state to the un-collapsed state by rotating along a circumference (6) of the drive shaft due to resistance from a liquid (7) in the bioreactor when agitation is performed.

2. Impeller system (1) according to claim 1, wherein the at least two impeller blades (5) are each independently connected to the drive shaft (3).

3. Impeller system (1) according to claim 2, wherein at least one of the at least two impeller blades (5) is independently attached to the drive shaft (3) with a ring (8, 29, 30), configured for rotation around the drive shaft, wherein the ring is configured for rotating from a first orientation on the drive shaft in the collapsed state (I) to a second orientation on the drive shaft in the un-collapsed state (II), such that the at least two impeller blades are positioned axisymmetrically around the drive shaft.

4. Impeller system (1) according to claim 3, wherein one of the ring (8, 29, 30) or a local circumference (9) of the drive shaft (3) at the axial location of the ring is provided with an engagement portion (10) and the other of the ring or the local circumference is provided with an engagement member (11), wherein the engagement portion and the engagement member are configured to engage each other when the ring has reached the second orientation, thereby preventing rotation of the ring past the second orientation.

5. Impeller system (1) according to any one of the preceding claims, wherein, in the collapsed state (I), the at least two impeller blades (5) are adjacent to each other (16) along the longitudinal axis (X), preferably in such a way, that contours (17) of the at least two impeller blades are aligned, when viewed along the longitudinal axis.

6. Impeller system (1) according to any one of the preceding claims, wherein radially outer edges (12) of the two or more impeller blades (5) are rounded in a main plane (13) of the impeller blade, wherein the rounded, radially outer edges (12) of the two or more impeller blades (5) preferably have a constant radius of curvature (r), and/or wherein radially outer edges (12) of the two or more impeller blades (5) are rounded in a plane (14) transversal to the main plane (13) of the impeller blade and the radially outer edges.

7. Impeller system (1) according to any one of the preceding claims, wherein the at least two impeller blades (5) in the collapsed state (I) are aligned along the longitudinal axis.

8. Impeller system (1) according to any one of the preceding claims, wherein the at least two impeller blades (5) in the collapsed state (I) establish a rotational angle with respect to each other about the longitudinal axis (X) that is less than 45 degrees, more preferably less than 30 degrees, and even more preferably less than 15 degrees, most preferably around 0 degrees.

9. Flexible container for bioreaction (15), comprising an impeller system (1) according to any one of the preceding claims, wherein the impeller system is arranged inside the flexible container for bioreaction, wherein the inside of the flexible container for bioreaction preferably is sterile to a sterility assurance level of at least 10-3 SAL.

10. Flexible container for bioreaction (15) according to claim 9, wherein the at least two impeller blades (5) are in the collapsed state (I).

11. Flexible container for bioreaction (15) according to any one of the claims 9 - 10, further comprising a sterility barrier encapsulating the flexible container, wherein the sterility barrier is optionally configured as a bag, pouch, or a tub with a sealed lid.

12. Bioreactor (2), comprising a drive motor (4) and an impeller system (1) according to any one of the claims 1 - 8 or a flexible container for bioreaction (15) according to any one of the claims 9 - 11, wherein the drive shaft (3) is connected to the drive motor (4).

13. Method of using an impeller system (1) according to any one of the claims 1 - 8, comprising the steps of:
- connecting the drive shaft (3) to the drive motor (4) of the bioreactor (2); and
- rotating the drive shaft around the longitudinal axis (X) of the drive shaft by the drive motor of the bioreactor, wherein the at least one of the at least two impeller blades (5) transitions from the collapsed state (I) to the un-collapsed state (II) by rotating along a circumference (6) of the drive shaft due to resistance from the liquid (7) in the bioreactor, for performing agitation of the liquid.

14. Method of manufacturing an impeller system (1) according to any one of the claims 1 - 8, comprising the step of:
- manufacturing a drive shaft (3) configured for being rotated in a rotational direction (R) around a longitudinal axis (X) of the drive shaft by a drive motor (4) of the bioreactor (2);
- manufacturing at least two impeller blades (5) for connection to the drive shaft, and for being rotated along with the drive shaft, wherein at least one of the at least two impeller blades is configured for transitioning from
a collapsed state (I), wherein the at least two impeller blades are not positioned axisymmetrically around the drive shaft to an un-collapsed state (II), for performing agitation, wherein the at least two impeller blades are positioned axisymmetrically around the drive shaft, wherein in the collapsed state (I) the at least two impeller blades (5) are preferably aligned along the longitudinal axis (X);
wherein the at least one of the at least two impeller blades transitions from the collapsed state to the un-collapsed state by rotating along a circumference (6) of the drive shaft due to resistance from a liquid (7) in the bioreactor when agitation is performed; and
- connecting the at least two impeller blades to the drive shaft.

15. Method according to claim 14, wherein manufacturing the at least two impeller blades (5) comprises 3D-printing at least one of the at least two impeller blades.

## Patentansprüche

1. Laufradsystem (1) zur Verwendung mit einem Bioreaktor (2), welches umfasst:
- eine Antriebswelle (3), die dafür ausgelegt ist, von einem Antriebsmotor (4) des Bioreaktors in einer Drehrichtung (R) um eine Längsachse (X) der Antriebswelle gedreht zu werden;
- mindestens zwei mit der Antriebswelle verbundene Laufradschaufeln (5), die dafür ausgelegt sind, zusammen mit der Antriebswelle gedreht zu werden, wenn die Antriebswelle gedreht wird, wobei die mindestens zwei Laufradschaufeln ausgelegt sind zum Übergehen aus
einem zusammengeklappten ersten Zustand (I), wobei die mindestens zwei Laufradschaufeln nicht achsensymmetrisch um die Antriebswelle positioniert sind, in
einen auseinandergeklappten zweiten Zustand (II) zum Ausführen einer Rührbewegung, wobei die mindestens zwei Laufradschaufeln achsensymmetrisch um die Antriebswelle positioniert sind,
**dadurch gekennzeichnet, dass** mindestens eine der mindestens zwei Laufradschaufeln durch Drehen entlang eines Umfangs (6) der Antriebswelle infolge des Widerstands von einer Flüssigkeit (7) im Bioreaktor aus dem zusammengeklappten Zustand in den auseinandergeklappten Zustand übergeht, wenn eine Rührbewegung ausgeführt wird.

2. Laufradsystem (1) nach Anspruch 1, wobei die mindestens zwei Laufradschaufeln (5) jeweils unabhängig mit der Antriebswelle (3) verbunden sind.

3. Laufradsystem (1) nach Anspruch 2, wobei mindestens eine der mindestens zwei Laufradschaufeln (5) unabhängig mit einem Ring (8, 29, 30) an der Antriebswelle (3) befestigt ist, der für eine Drehung um die Antriebswelle ausgelegt ist, wobei der Ring dafür ausgelegt ist, sich von einer ersten Ausrichtung auf der Antriebswelle im zusammengeklappten Zustand (I) zu einer zweiten Ausrichtung auf der Antriebswelle im auseinandergeklappten Zustand (II) zu drehen, so dass die mindestens zwei Laufradschaufeln achsensymmetrisch um die Antriebswelle positioniert sind.

4. Laufradsystem (1) nach Anspruch 3, wobei einer von dem Ring (8, 29, 30) und einem lokalen Umfang (9) der Antriebswelle (3) an der axialen Position des Ringes mit einem Eingriffsabschnitt (10) versehen ist und der andere von dem Ring und dem lokalen Umfang mit einem Eingriffselement (11) versehen ist, wobei der Eingriffsabschnitt und das Eingriffselement dafür ausgelegt sind, miteinander in Eingriff zu gelangen, wenn der Ring die zweite Ausrichtung erreicht hat, und dadurch eine Drehung des Ringes an der zweiten Ausrichtung vorbei zu verhindern.

5. Laufradsystem (1) nach einem der vorhergehenden Ansprüche, wobei im zusammengeklappten Zustand (I) die mindestens zwei Laufradschaufeln (5) einander entlang der Längsachse (X) benachbart sind (16), vorzugsweise auf eine solche Weise, dass Konturen (17) der mindestens zwei Laufradschaufeln zueinander ausgerichtet sind, wenn sie entlang der Längsachse betrachtet werden.

6. Laufradsystem (1) nach einem der vorhergehenden Ansprüche, wobei radial äußere Ränder (12) der zwei oder mehr Laufradschaufeln (5) in einer Hauptebene (13) der Laufradschaufel abgerundet sind, wobei die abgerundeten, radial äußeren Ränder (12) der zwei oder mehr Laufradschaufeln (5) vorzugsweise einen konstanten Krümmungsradius (r) aufweisen, und/oder wobei radial äußere Ränder (12) der zwei oder mehr Laufradschaufeln (5) in einer Ebene (14) abgerundet sind, die quer zur Hauptebene (13) der Laufradschaufel und den radial äußeren Rändern verläuft.

7. Laufradsystem (1) nach einem der vorhergehenden Ansprüche, wobei die mindestens zwei Laufradschaufeln (5) im zusammengeklappten Zustand (I) entlang der Längsachse ausgerichtet sind.

8. Laufradsystem (1) nach einem der vorhergehenden Ansprüche, wobei die mindestens zwei Laufradschaufeln (5) im zusammengeklappten Zustand (I) einen Drehwinkel um die Längsachse (X) relativ zueinander aufweisen, welcher kleiner als 45 Grad, stärker bevorzugt kleiner als 30 Grad und sogar noch stärker bevorzugt kleiner als 15 Grad ist und am meisten bevorzugt etwa 0 Grad beträgt.

9. Flexibler Behälter zur Bioreaktion (15), welcher ein Laufradsystem (1) nach einem der vorhergehenden Ansprüche umfasst, wobei das Laufradsystem im Inneren des flexiblen Behälters zur Bioreaktion angeordnet ist, wobei das Innere des flexiblen Behälters zur Bioreaktion vorzugsweise steril mit einem Sterilitätssicherheitsniveau von mindestens 10-3 SAL ist.

10. Flexibler Behälter zur Bioreaktion (15) nach Anspruch 9, wobei sich die mindestens zwei Laufradschaufeln (5) im zusammengeklappten Zustand (I) befinden.

11. Flexibler Behälter zur Bioreaktion (15) nach einem der Ansprüche 9 - 10, welcher ferner eine Sterilitätsbarriere umfasst, die den flexiblen Behälter umhüllt, wobei die Sterilitätsbarriere optional als eine Tasche, ein Beutel oder eine Wanne mit einem abgedichteten Deckel ausgebildet ist.

12. Biorektor (2), welcher einen Antriebsmotor (4) und ein Laufradsystem (1) nach einem der Ansprüche 1 - 8 oder einen flexiblen Behälter zur Bioreaktion (15) nach einem der Ansprüche 9 - 11 umfasst, wobei die Antriebswelle (3) mit dem Antriebsmotor (4) verbunden ist.

13. Verfahren zur Verwendung eines Laufradsystems (1) nach einem der Ansprüche 1 - 8, welches die Schritte umfasst:
- Verbinden der Antriebswelle (3) mit dem Antriebsmotor (4) des Bioreaktors (2); und
- Drehen der Antriebswelle um die Längsachse (X) der Antriebswelle durch den Antriebsmotor des Bioreaktors, wobei die mindestens eine der mindestens zwei Laufradschaufeln (5) durch Drehen entlang eines Umfangs (6) der Antriebswelle infolge des Widerstands von der Flüssigkeit (7) im Biorektor aus dem zusammengeklappten Zustand (I) in den auseinandergeklappten Zustand (II) übergeht, um ein Rühren der Flüssigkeit auszuführen.

14. Verfahren zur Herstellung eines Laufradsystems (1) nach einem der Ansprüche 1 - 8, welches die Schritte umfasst:
- Herstellen einer Antriebswelle (3), die dafür ausgelegt ist, von einem Antriebsmotor (4) des Bioreaktors (2) in einer Drehrichtung (R) um eine Längsachse (X) der Antriebswelle gedreht zu werden;
- Herstellen mindestens zweier Laufradschaufeln (5) zur Verbindung mit der Antriebswelle und um zusammen mit der Antriebswelle gedreht zu werden, wobei mindestens eine der mindestens zwei Laufradschaufeln ausgelegt ist zum Übergehen aus
einem zusammengeklappten Zustand (I), wobei die mindestens zwei Laufradschaufeln nicht achsensymmetrisch um die Antriebswelle positioniert sind, in einen auseinandergeklappten Zustand (II) zum Ausführen einer Rührbewegung, wobei die mindestens zwei Laufradschaufeln achsensymmetrisch um die Antriebswelle positioniert sind, wobei im zusammengeklappten Zustand (I) die mindestens zwei Laufradschaufeln (5) vorzugsweise entlang der Längsachse (X) ausgerichtet sind;
wobei die mindestens eine der mindestens zwei Laufradschaufeln durch Drehen entlang eines Umfangs (6) der Antriebswelle infolge des Widerstands von einer Flüssigkeit (7) im Bioreaktor aus dem zusammengeklappten Zustand in den auseinandergeklappten Zustand übergeht, wenn eine Rührbewegung ausgeführt wird; und
- Verbinden der mindestens zwei Laufradschaufeln mit der Antriebswelle.

15. Verfahren nach Anspruch 14, wobei das Herstellen der mindestens zwei Laufradschaufeln (5) das 3D-Drucken mindestens einer der mindestens zwei Laufradschaufeln umfasst.

## Revendications

1. Système de roue (1) pour une utilisation avec un bioréacteur (2), comprenant :
- un arbre d'entraînement (3) configuré pour tourner dans une direction de rotation (R) autour d'un axe longitudinal (X) de l'arbre d'entraînement par un moteur d'entraînement (4) du bioréacteur ;
- au moins deux aubes de roue (5) reliées à l'arbre d'entraînement, configurées pour tourner avec l'arbre d'entraînement lorsque l'arbre d'entraînement tourne, où les au moins deux aubes de roue sont configurées pour passer
d'un premier état replié (I), dans lequel les au moins deux aubes de roue ne sont pas positionnées de manière axisymétrique autour de l'arbre d'entraînement,
à un état non replié (II), pour réaliser une agitation, dans lequel les au moins deux aubes de roue sont positionnées de manière axisymétrique autour de l'arbre d'entraînement,
**caractérisé en ce qu'**au moins l'une des au moins deux aubes de roue passe de l'état replié à l'état non replié en tournant le long d'une circonférence (6) de l'arbre d'entraînement en raison de la résistance d'un liquide (7) dans le bioréacteur lorsque l'agitation est réalisée.

2. Système de roue (1) selon la revendication 1, dans lequel les au moins deux aubes de roue (5) sont chacune reliées indépendamment à l'arbre d'entraînement (3).

3. Système de roue (1) selon la revendication 2, dans lequel au moins l'une des au moins deux aubes de roue (5) est fixée indépendamment à l'arbre d'entraînement (3) avec une bague (8, 29, 30), configurée pour la rotation autour de l'arbre d'entraînement, où la bague est configurée pour tourner d'une première orientation sur l'arbre d'entraînement dans l'état replié (I) à une seconde orientation sur l'arbre d'entraînement dans l'état non replié (II), de sorte que les au moins deux aubes de roue soient positionnées de manière axisymétrique autour de l'arbre d'entraînement.

4. Système de roue (1) selon la revendication 3, dans lequel l'une de la bague (8, 29, 30) ou d'une circonférence locale (9) de l'arbre d'entraînement (3) à l'emplacement axial de la bague est munie d'une partie d'engagement (10) et l'autre de la bague ou de la circonférence locale est munie d'un élément d'engagement (11), où la partie d'engagement et l'élément d'engagement sont configurés pour s'engager l'un dans l'autre lorsque la bague a atteint la seconde orientation, empêchant ainsi la rotation de la bague au-delà de la seconde orientation.

5. Système de roue (1) selon l'une quelconque des revendications précédentes, dans lequel, dans l'état replié (I), les au moins deux aubes de roue (5) sont adjacentes l'une à l'autre (16) le long de l'axe longitudinal (X), de préférence de manière à ce que les contours (17) des au moins deux aubes de roue soient alignés, lorsqu'ils sont vus le long de l'axe longitudinal.

6. Système de roue (1) selon l'une quelconque des revendications précédentes, dans lequel des bords radialement externes (12) des deux aubes de roue (5) ou plus sont arrondis dans un plan principal (13) de l'aube de roue, dans lequel les bords radialement externes, arrondis (12) des deux aubes de roue (5) ou plus ont de préférence un rayon de courbure constant (r), et/ou dans lequel les bords radialement externes (12) des deux aubes de roue (5) ou plus sont arrondis dans un plan (14) transversal au plan principal (13) de l'aube de roue et aux bords radialement externes.

7. Système de roue (1) selon l'une quelconque des revendications précédentes, dans lequel les au moins deux aubes de roue (5) dans l'état replié (I) sont alignées le long de l'axe longitudinal.

8. Système de roue (1) selon l'une quelconque des revendications précédentes, dans lequel les au moins deux aubes de roue (5) dans l'état replié (I) établissent un angle de rotation l'une par rapport à l'autre autour de l'axe longitudinal (X) qui est inférieur à 45 degrés, plus préférablement inférieur à 30 degrés, et encore plus préférablement inférieur à 15 degrés, idéalement d'environ 0 degré.

9. Récipient souple pour une bioréaction (15), comprenant un système de roue (1) selon l'une quelconque des revendications précédentes, dans lequel le système de roue est agencé à l'intérieur du récipient souple pour une bioréaction, dans lequel l'intérieur du récipient souple pour une bioréaction est de préférence stérile à un niveau d'assurance de stérilité d'au moins 10-3 SAL.

10. Récipient souple pour une bioréaction (15) selon la revendication 9, dans lequel les au moins deux aubes de roue (5) sont dans l'état replié (I).

11. Récipient souple pour une bioréaction (15) selon l'une quelconque des revendications 9 et 10, comprenant en outre une barrière de stérilité encapsulant le récipient souple, où la barrière de stérilité est éventuellement configurée comme un sac, une poche ou un bac avec un couvercle scellé.

12. Bioréacteur (2), comprenant un moteur d'entraînement (4) et un système de roue (1) selon l'une quelconque des revendications 1 à 8 ou un récipient souple pour une bioréaction (15) selon l'une quelconque des revendications 9 à 11, dans lequel l'arbre d'entraînement (3) est relié au moteur d'entraînement (4).

13. Procédé d'utilisation d'un système de roue (1) selon l'une quelconque des revendications 1 à 8, comprenant les étapes :
- de liaison de l'arbre d'entraînement (3) au moteur d'entraînement (4) du bioréacteur (2) ; et
- de rotation de l'arbre d'entraînement autour de l'axe longitudinal (X) de l'arbre d'entraînement par le moteur d'entraînement du bioréacteur, où l'au moins une des au moins deux aubes de roue (5) passe de l'état replié (I) à l'état non replié (II) en tournant le long d'une circonférence (6) de l'arbre d'entraînement en raison de la résistance du liquide (7) dans le bioréacteur, pour réaliser l'agitation du liquide.

14. Procédé de fabrication d'un système de roue (1) selon l'une quelconque des revendications 1 à 8, comprenant l'étape :
- de fabrication d'un arbre d'entraînement (3) configuré pour tourner dans une direction de rotation (R) autour d'un axe longitudinal (X) de l'arbre d'entraînement par un moteur d'entraînement (4) du bioréacteur (2) ;
- de fabrication d'au moins deux aubes de roue (5) pour la liaison à l'arbre d'entraînement et pour tourner avec l'arbre d'entraînement, où au moins l'une des au moins deux aubes de roue est configurée pour passer
d'un état replié (I), dans lequel les au moins deux aubes de roue ne sont pas positionnées de manière axisymétrique autour de l'arbre d'entraînement à un état non replié (II), pour réaliser une agitation, dans lequel les au moins deux aubes de roue sont positionnées de manière axisymétrique autour de l'arbre d'entraînement, où, dans l'état replié (I), les au moins deux aubes de roue (5) sont de préférence alignées le long de l'axe longitudinal (X) ;
dans lequel l'au moins une des au moins deux aubes de roue passe de l'état replié à l'état non replié en tournant le long d'une circonférence (6) de l'arbre d'entraînement en raison de la résistance d'un liquide (7) dans le bioréacteur lorsque l'agitation est réalisée ; et
- de liaison des au moins deux aubes de roue à l'arbre d'entraînement.

15. Procédé selon la revendication 14, dans lequel la fabrication des au moins deux aubes de roue (5) comprend l'impression 3D d'au moins l'une des au moins deux aubes de roue.
